Europäisches Patentamt

European Patent Office

Office européen des brevets

(11) Publication number: **0 218 386**
A1

# EUROPEAN PATENT APPLICATION

(21) Application number: 86307095.9

(22) Date of filing: 15.09.86

(51) Int. Cl.⁴: **A 01 N 63/04,** C 12 N 1/14
// (C12N1/14, C12R1:645)

(30) Priority: 16.09.85 US 776209

(43) Date of publication of application: 15.04.87
Bulletin 87/16

(84) Designated Contracting States: DE FR GB IT

(71) Applicant: **Her Majesty in Right of Canada as
represented by the Minister of Agriculture Canada,
930 Carling Avenue, Ottawa Ontario (CA)**

(72) Inventor: **Mortensen, Knud, 2312 Robinson Street,
Regina Saskatchewan (CA)**

(74) Representative: **Holmes, Michael John et al, Frank B.
Dehn & Co. European Patent Attorneys Imperial
House 15-19 Kingsway, London, WC2B 6UZ, (GB)**

(54) **Control of round-leaved mallow and velvetleaf weeds with C. gloeosporioides.**

(57) Round-leaved mallow and velvetleaf weeds are controlled
by inoculating the weeds with an amount of the fungus Col-
letotrichum gloeosporioides (Penz.) Sacc, American Type Cul-
ture Number 20767, sufficient to infect and kill the weeds.

- 1 -

## Control of Round-Leaved Mallow and Velvetleaf Weeds with C. Gloeosporioides

### Background of the Invention

This invention relates to a process for biological control of weeds, particularly round-leaved mallow and velvetleaf weeds.

Endemic pathogens have been used for biological control of weeds and for instance, Daniel et. al., U.S. Patent 3,849,104, issued Nov. 19, 1974, describes a method for controlling northern jointvetch by inoculation with a species of the fungus Colletotrichum.

Other problem weeds include round-leaved mallow and velvetleaf. Round-leaved mallow is a common weed of farm yards and gardens, but recently is becoming a troublesome weed in field crops in western Canada. There has not been an adequate method to control round-leaved mallow and in 1984 it was necessary to plough flax fields under, due to round-leaved mallow infestation.

Velvetleaf is a serious weed in soybean crops in Ontario, Canada and in the mid-west of the United States. It has not been possible to control velvetleaf with available herbicides.

### Summary of the Invention

According to this invention it has been found that a newly isolated species of the fungus Colletotrichum is uniquely capable of controlling both round-leaved mallow and velvetleaf weeds. The effective species is Colletotrichum gloeosporioides, more particularly strain (Penz.) Sacc., A.T.C.C. No. 20767.

Detailed Description

The above species was originally isolated from seedling blight occurring on round-leaved mallow (Malva pusilla Sm.) in greenhouse tests. Later the disease, appearing as anthracnose symptoms, was observed on round-leaved mallow plants under natural conditions from several locations in Saskatchewan, Canada. Under natural conditions the disease does not develop into epidemic proportions in stands of round-leaved mallow until late in the growing season. However, under greenhouse conditions round-leaved mallow plants were severely attacked and often totally killed when inoculated with this pathogen.

Isolation of C. gloeosporioides was done by plating out sections of diseased plant material (surfaced sterilized for 1 min, rinsed in sterile water) on potato dextrose agar (PDA). Round-leaved mallow and test plants used in these experiments were grown from seeds in a sandy loam soil mixed (3:1) with sphagnum (peat moss) and grown on greenhouse benches at a temperature of 18 - 24°C with a 14 hour day extended with incandescent and fluorescent light. Seeds used were obtained from Regina Research Station seed stock, from research scientists and from seed supply stores. If not specified, plants were inoculated in the well developed seedling stage (2 - 4 weeks after planting, depending on the plant species). Spores of C. gloeosporioides used for inoculating test plants were produced on potato dextrose agar incubated in an incubator (Conviron, I18L) for 6 - 8 days at 24°C with a 12 hour light cycle of fluorescent light (275 microeinstein per m sq. per sec.). Spores (conidia) produced in acervuli on potato dextrose agar were transferred to distilled water by means of a bacterial loop. The spore suspension (concentration determined by counting spores in a haemocytometer) was sprayed on test plants by means of an air brush (Paasche Airbrush (Canada) Ltd, Type H-5) operated with a constant air pressure (30 lb/sq. inch) until runoff. Concentrations of spore suspensions were normally 2-5 million spores/ml. After inoculation, test plants were kept in a mist chamber for three days to assure good infection. The mist chamber was constructed on a greenhouse bench and enclosed with transparent polyethylene, the day/night cycle and temperature range in the mist chamber being similar to that of the greenhouse. Mist was supplied by a cool water mister operating 2 minutes out of every 12, creating enough moisture in the chamber to keep plants wet without run-off. After leaving the mist chamber, inoculated plants were kept on greenhouse benches for 15 to 20 days (under regular inspection) until a final disease rating was done using a scale from 0-9:

0: Totally resistant (immune), no visible symptoms.

1: Few restricted lesions developing, covering less than 2 % of plant surfaces.

2: Few restricted lesions developing, covering up to 5 % of plant surfaces. Lesions in 1 and 2 are very small (.5 mm diameter) and do not affect plant development.

3: Restricted lesions developing, covering up to 10 % of plant surfaces.

4: Restricted lesions developing, covering up to 15 % of plant surfaces. Lesions in 3 and 4 are small (2 mm diameter) and do not cause wilting of plant material outside lesions, thus not affecting plant development seriously.

5: Lesion development girdling smaller branches and leaf petioles, causing wilt of up to 20 % of plant material.

6: Lesion development girdling smaller branches and leaf petioles, causing wilt of up to 50 % of plant material.

7: Lesion development girdling medium size stem branches, causing wilt of up to 75 % of plant material.

8: Lesion development girdling main stem branches, causing wilt of up to 90 % of plant material.

9: Lesion development girdling all stem branches, causing wilt of more than 90 % of plant material.

In a preliminary test it was found that an 18-24 hour mist period after inoculation, where plants were kept wet without runoff, was required to obtain good infection of C. gloeosporioides on round-leaved mallow. First sign of infection is visible 5-6 days after inoculation. It appears as a dark sunken lesion on stems and leaf petioles. After 2 weeks, the lesions (2-5 mm diameter) turn greyish in the center with an almost black margin. Later lesions often coalesce and sever entire stem or leaf petioles resulting in wilting of the stem above the lesion. In the center of the lesion small pinkish dots are visible. These are the acervuli in which oblong (10 X 6 μm) hyalin conidia are produced readily under moist conditions.

Example 1

C. gloeosporioides was isolated from diseased round-leaved mallow plants from four different locations in Saskatchewan, Canada, namely Antler, Estuary, Lockwood and Regina. These were prepared and tested in accordance with the procedures described above and the pathogenicity was determined on round-leaved mallow at different growth stages from the young seedling stage (2 weeks old) to fully mature plants (10 to 12 weeks old). The results are shown in Table I below:

Table 1. Pathogenicity of C. gloeosporioides from four different locations in Saskatchewan on round-leaved mallow at varying plant stages.

| Origin of isolate | Inoculum conc. (mill. spores per ml.) | Age of r.l.m. plants at inoculation (weeks) | | | | | |
|---|---|---|---|---|---|---|---|
| | | 2 | 3 | 7 | 9 | 10 | 12 |
| Antler | 8.5 | 9* | 9 | 9 | 9 | 7 | 7 |
| Estuary | 5.5 | 9 | 9 | 8 | 8 | 7 | 7 |
| Lockwood | 8.5 | 9 | 9 | 9 | 9 | 8 | 7 |
| Regina | 7.6 | 9 | 6 | 6 | 6 | 6 | 6 |

* Disease rating 17 days after inoculation (scale: 0 - 9).

Example 2

Round-leaved mallow seeds collected from four locations in Saskatchewan (Regina, Raymore, Strasbourg and Mossbank), and two locations in Manitoba (Manitou and Steinbach) and common mallow (Malva neglecta) from Summerland, B.C. were planted and grown under greenhouse condition and inoculated with the Regina isolate of C. gloeosporioides. A.T.C.C. No. 20767.

(a) The round-leaved mallow plants were inoculated twice with 10 days interval (inoculum concentration: 2.8 mill. and 9.9 million spores/ml) and rated 14 days after the last inoculation. The results were:

| Rep. | 1 | 2 | Mean | Total No. of plants tested |
|---|---|---|---|---|
| Regina, Sk. | 8 | 8 | 8 | 5 |
| Raymore, Sk. | 8 | 8 | 8 | 6 |
| Strasbourg, Sk. | 6 | 6 | 6 | 3 |
| Mossbank, Sk. | 8 | 8 | 8 | 9 |
| Manitou, Man. | 9 | 9 | 9 | 5 |
| Steinbach, Man. | 9 | - | 9 | 2 |

(b) The procedure of part (a) was repeated but using an inoculum concentration of 19 million spores/ml and rated 17 days after inoculation. The results were:

| Rep. | 1 | 2 | 3 | Mean* | Total No. of plants tested |
|------|---|---|---|-------|----------------------------|
| Regina, Sk. | 8 | 8 | 8 | 8 | 4 |
| Raymore, Sk. | 6 | 7 | 4 | 5.7+-0.9 | 6 |
| Strasbourg, Sk. | 4 | - | - | 4 | 1 |
| Mossbank, Sk. | 6 | - | - | 6 | 3 |
| Manitou, Man. | 9 | 9 | 9 | 9 | 9 |
| Steinbach, Man. | 9 | 9 | 8 | 8.7+-0.3 | 7 |

* Mean +- standard error of mean.

(c) The procedure of part (a) was repeated but using an inoculum concentration of 6.3 million spores/ml and rated 20 days after inoculation. The results were:

| Rep. | 1 | 2 | 3 | 4 | Mean* | Total No. of plants tested |
|------|---|---|---|---|-------|----------------------------|
| Regina, Sk. | 4 | 8 | 8 | - | 6.7+-1.3 | 11 |
| Raymore, Sk. | 8 | 8 | 8 | 7 | 7.8+-0.3 | 9 |
| Strasbourg, Sk. | 4 | 6 | 6 | 6 | 5.5+-0.5 | 14 |
| Mossbank, Sk. | 5 | 7 | 5 | 5 | 5.5+-0.5 | 13 |
| Manitou, Man. | 9 | 4 | 4 | - | 5.7+-1.7 | 5 |
| Steinbach, Man. | 8 | 8 | 9 | 7 | 8.0+-0.4 | 13 |
| Summerland, B.C.** | 4 | 4 | 4 | 4 | 4.0 +-0.0 | 15 |

*Mean +- standard of error of mean, ** Common mallow.

Example 3

Using the procedures described above, pathogenicity tests were conducted on a range of test plant species inoculated with C. gloeosporioides, A.T.C.C. No. 20767. The results are shown in Table 2 below:

0218386

## Table 2. Pathogenicity test of <u>Colletotrichum</u> <u>gloeosporioides</u> on selected plant species

| Plant Species tested | No. of plants tested | Disease scale (0-9) |
|---|---|---|
| **Malvaceae:** | | |
| <u>Malva</u> <u>pusilla</u> L. (round-leaved mallow) | 18 | 7-9 |
| <u>M</u>. <u>alcea</u> L. | 5 | 5 |
| <u>M</u>. <u>alcea</u> Fastigiata | 19 | 7 |
| <u>M</u>. <u>neglecta</u> L. (common mallow) | 2 | 7 |
| <u>M</u>. <u>moschata</u> L. (musk mallow) | 4 | 4 |
| <u>M</u>. <u>sylvestris</u> L. | 1 | 2 |
| <u>Althaea</u> <u>rosea</u> Cav. (hollyhock) | | |
| cv. Chater's Double mixed | 6 | 3 |
| cv. Majorette Mixed | 2 | 0 |
| cv. Summer Carnival | 2 | 0 |
| <u>Sidalceae</u> sp. TM Special Hybrid | 1 | 0 |
| <u>Anoda</u> <u>cristata</u> Schlecht (spurred anoda) | 28 | 0 |
| <u>Sida</u> <u>spinosa</u> L. (prickly sida) | 18 | 0 |
| <u>Abutilon</u> <u>theophrasti</u> Medic (velvetleaf) | 10 | 6-7 |
| <u>Pavonia</u> <u>hastata</u> Cav. | 3 | 0 |
| <u>Malope</u> <u>trifida</u> Cav. | 7 | 4 |
| <u>Gossypium</u> <u>hirsutum</u> L. (cotton) | | |
| cv. Pima S-5 | 4 | 0 |
| cv. DPL - 61 | 5 | 0 |
| cv. Stoneville 213 | 6 | 0 |
| <u>Lagunaria</u> <u>patersonii</u> Don. | 1 | 0 |
| <u>Hibiscus</u> <u>trionum</u> L. (Venice mallow) | 12 | 4 |
| <u>H</u>. <u>esculentus</u> L. (okra) cv. Perkin's Mammoth Longpod | 9 | 0 |
| <u>H</u>. <u>sabdariffa</u> L. | 1 | 0 |
| **Caryophyllaceae:** | | |
| <u>Saponaria</u> <u>vaccaria</u> L. (cow cockle) | 21 | 0 |

(Table 2 - continued)

Dianthus caryophyllus L. (carnation)
                                    cv. Gaiety
                                        Double Mixed    21    0
Compositae:
    Helianthus annuus L. (sunflower)                    11    0
    Carthamus tinctorius L. (safflower)
                                    cv. S208             5    1
    Lactuca sativa L. (lettuce) cv. Red Salad
                                        Bowl            63    0
Leguminoseae:
    Medicago sativa L. (alfalfa) cv. Algonquin          24    0
    Lens culinaris Medic. (lentils)                     12    0
    Phaseolus vulgaris L. cv. Pencil Pod
                                    Black Wax            7    0
    Pisium sativum L. (field peas) cv. Century           5    0
    Vicia faba L. (fababeans) cv. Herz Freyea            9    0
    Glycine max (L.) Merrill (soybeans)
                                    cv. Kentland        13    0
                                    cv. Maple Arrow      9    0
                                    cv. Corsoy 79       10    0
    Aeschynomene virginica (L.)B.S.P.
                            (northern jointvetch)        6    0
Linaceae:
    Linum usitatissimum L. (flax) cv. Noralta           34    0
Cruciferae:
    Brassica napus L. (rapeseed) cv. Regent             30    0
    B. campestris L. (rapeseed) cv. Candle              79    0
    B. hirta Moench (white mustard)                     47    1
Solanaceae:
    Lycopersicon esculentum Mill. (tomato)
                                    cv. Bonny Best      12    0
Chenopodiaceae:
    Beta vulgaris L. (sugarbeets)
                                    cv. Detroit Dark Red 28   0

0218386

(Table 2 - continued)

Polygonaceae:

    _Fagopyrum_ _esculentum_ Moench (buckwheat)    3     0

Umbelliferae:

    _Daucus_ _carota_ L. (carrots)

                cv. Early Cross Hybrid   36    0

Cucurbitaceae:

    _Cucumis_ _sativus_ L. (cucumber)

                cv. Chicago Pickling   5     0

Graminae:

    _Triticum_ _aestivum_ L. (wheat) cv. Nepawa   13    0

    _Hordeum_ _vulgare_ L. (barley) cv. Betzes   16    0

    _Avena_ _sativa_ L. (oats) cv. Cavell   13    0

    _Secale_ _cereale_ L. (rye) cv. Frontier   10    0

    _Zea_ _mays_ L. (corn) cv. Gold Bantam   3     0

    _Phalaris_ _canariensis_ (canary grass)

                cv. Keet   50    0

-----------------------------------------------------------

\* 0: no symptoms observed, 9: more than 90 per cent of test plants killed.

The above results showed that only species in the Malvaceae family were susceptible except for very small restricted lesion development on cotyledons of safflower (_Carthamus_ _tinctorius_) and white mustard (_Brassica_ _hirta_) which did not harm the plants. Only a few species in the _Malva_ genus plus velvetleaf (_Abutilon_ _theophrasti_ Medic.) were susceptible (rated 5 or higher on the 0-9 disease scale). Restricted leaf lesions were observed on _M._ _moschata_, _M._ _sylvestris_, _Altheae_ _rosea_, _Malope_ _trifida_ and _Hibiscus_ _trionum_, but none of these ornamental plant species were damaged seriously and under greenhouse conditions they outgrew the disease. All three cultivars of cotton (_Gossypium_ _hirsutum_ L.) included in the tests were immune.

Example 4

Tests were conducted to compare the C. gloeosporioides, A.T.C.C. No. 20767, of this invention with two known species of Colletotrichum, C. gloeosporioides (sp. aeschynomene and C. malvarum).

C. gloeosporioides from round-leaved mallow grows well on several agar media. On PDA it produces a white greyish mycelium, which very quickly turns black and pinkish masses of spores are produced readily in acervuli on young mycelium, but to a lesser extent in older cultures. C. gloeosporioides f. sp. aeschynomene grew slightly faster on PDA, produced spores abundantly, but did not develop as much dark mycelium. C. malvarum was similar to C. gloeosporioides from round-leaved mallow on PDA, only acervuli with spores were much smaller. Conidia sizes (means of measurements on 15 spores per fungi) were:

C. gloeosporioides, round-leaved mallow
10.8+-0.4 by 6.0+-0.3 μm, range: 8.1-14.1 by 4.0-8.1 μm.
C. gloeosporioides f. sp. aeschynomene
14.5+-0.3 by 4.7 +-0.2 μm, range: 12.1-16.2 by 4.0-6.1 μm.
C. malvarum
12.4+-0.3 by 5.6+-0.1 μm, range: 10.1-14.1 by 5.1-6.1 μm.
Thus the three fungi can be distinguished morphologically in cultures on PDA. As shown in Table 2, C. gloeosporioides, round-leaved mallow, was not pathogenic on northern jointvetch and prickly sida, the hosts of C. gloeosporioides f. sp. aeschynomene and C. malvarum, respectively. Both C. gloeosporioides f. sp. aeschynomene and C. malvarum were non-pathogenic on round-leaved mallow.

According to Daniel et al., "Biological control of northern jointvetch in rice with an endemic fungal disease", Weed Science 21: 303-307 (1973), C. gloeosporioides f. sp. aeschynomene was pathogenic only on northern jointvetch and Indian vetch but Malvaceae species tested (cotton, okra) were immune. That indicates that the host range of C. gloeosporioides f. sp. aeschynomene and C. gloeosporioides from round-leaved mallow does not overlap.

According to Kirkpatrick et. al., "Potential of Colletotrichum malvarum for biological control of prickly sida", Plant Disease 66: 323-325, (1982), C. malvarum was pathogenic only on prickly sida and Althaea rosea (hollyhock) and immune to five other Malvaceae species (Abutilon theophrasti, Anoda cristata, Gyssypium hirsutum, Hibiscus esculentus and H. trionum). Hollyhock is the only species that showed some susceptibility to both C. malvarum and C. gloeosporioides from round-leaved mallow.

Example 5

The purpose of this experiment was to see if C. gloeosporioides A.T.C.C. No. 20767 a) can be cultured on velvetleaf for successive generations and b) its pathogenicity is stable or can a strain more adapted to velvetleaf possibly be selected. Typical lesions of C. gloeosporioides (round-leaved mallow) occur as stem lesions, mainly at the base of the stems, but occur quite frequently on upper stem parts also. A few infections observed on leaves, were restricted and did not cause serious harm to the plants.

The original inoculum of C. gloeosporioides (round-leaved mallow) for velvetleaf was bulked from several isolations from infected round-leaved mallow plants. This was done to have as much variation in the culture as possible. After incubation of inoculated velvetleaf plants, several isolations were made from developed lesions (3 pots, 10 plants) and spores from these isolations cultured on PDA (C. gleosporioides velvetleaf 1st generation) were bulked in a spore suspension (heavy, concentration not determined) and sprayed on 3 pots of new velvet-leaf plants. Isolations were made from lesions on 2 plants per pot, spores from these isolations (C.

gloeosporioides velvetleaf 2nd generation) were bulked and inoculated on a new set of velvetleaf plants (10 pots, 65 plants). From the 2nd generation lesions isolations were made from several lesions at the base of stems on plants from 2 pots, and from several upper stem lesions on plants from two other pots. Spores from each isolation (base and top lesions) (C. gloeosporioides 3rd generation) were bulked and inoculated on a new set of velvetleaf plants (3 pots, 4-10 plants per pot). After each inoculation, isolations from lesions on two plants per pot per culture were made. Spores from all isolations of a culture were bulked in a spore suspension and inoculated on a new set of velvetleaf plants (3 pots of 4-10 plants). This was repeated for 6 more generations of the two cultures (originating from base and top lesions). Along with these inoculations (from the fourth generation on) C. gloeosporioides (round-leaved mallow) was inoculated on one pot of round-leaved mallow and a set of velvetleaf plants. C. gloeosporioides (round-leaved mallow) spores isolated from the round-leaved mallow plants were then used in the following generation. This was done to have a direct comparison with the cultures cultured on velvetleaf. Seventeen to 20 days after each inoculation a disease rating was done on the inoculated velvetleaf plants in a pot using the 0-9 scale. Results of the above are shown in Table 3. The two cultures (originating from base and top lesions) were similar and, therefore, lumped, thus the data for C. gloeosporioides velvetleaf and C. gloeosporioides round-leaved mallow in Table 3 are based on six and three replications, respectively.

0218386

Table 3. C. gloeosporioides (round-leaved mallow) cultured on velvetleaf for 9 generations under greenhouse conditions.

| Number of inoculations (generations) | C. gloeosporioides velvetleaf | | C. gloeosporioides round-leaved mallow | |
|---|---|---|---|---|
| | No. of plants tested | Disease rating (0-9)* | No. of plants tested | Disease rating (0-9)* |
| 1st generation | 26 | 7.7 (7-8) | – | |
| 2nd generation | 65 | 7.6 (5-9) | – | |
| 3rd generation | 44 | 7.2 (5-9) | – | |
| 4th generation | 42 | 7.3 (5-8) | 19 | 7.0 (6-8) |
| 5th generation | 46 | 7.6 (7-9) | 28 | 7.3 (7-8) |
| 6th generation | 50 | 6.0 (5-7) | 20 | 6.3 (5-7) |
| 7th generation | 45 | 7.0 (6-9) | 33 | 6.3 (6-7) |
| 8th generation | 42 | 7.2 (6-8) | 25 | 7.7 (7-9) |
| 9th generation | 39 | 7.3 (6-8) | 13 | 7.0 (7-7) |

* Average of 3 pots in 1st generation, 10 pots in 2nd generation, 6 pots in 3rd to 9th generations.
** Average of 3 pots.

The results in Table 3 indicate that there were no differences in pathogenicity between cultures on velvetleaf for 9 generations and a culture cultured on round-leaved mallow. A test done at the end of the experiment, where 3 pots (24 plants) of round-leaved mallow were inoculated with C. gloeosporioides (round-leaved mallow) and another 3 pots (33 plants) of round-leaved mallow with C. gloeosporioides velvetleaf 10th generation, resulted in a disease rating of 6.3 (range: 5-8) and 8.7 (range: 8-9) for C. gloeosporioides (round-leaved mallow) and C. gloeosporioides (velvetleaf) 10th generation, respectively. This would indicate that the pathogenicity of C. gloeosporioides from round-leaved mallow is stable on both of these host plants.

- 14 -

CLAIMS:

1.   A process for controlling round-leaved mallow or velvetleaf weeds comprising inoculating the weeds with an amount of the fungus <u>Colletotrichum gloeosporioides</u> Penz. Sacc., ATCC 20767 sufficient to infect and kill the weeds.

2.   <u>Colletotrichum gloeosporioides</u> Penz. Sacc., ATCC 20767 and mutants thereof capable of infecting and killing round-leaved mallow and/or velvetleaf weeds.

European Patent
Office

0218386
Application number

**EUROPEAN SEARCH REPORT**

EP  86 30 7095

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A,D | US-A-3 849 104  (J.T.DANIEL et al.) | 1,2 | A 01 N    63/04<br>C 12 N     1/14 //<br>(C 12 N     1/14<br>C 12 R     1:645) |
| | --- | | |
| A | US-A-3 999 973  (G.E.TEMPLETON) | 1,2 | |
| | ----- | | |

TECHNICAL FIELDS
SEARCHED (Int. Cl.4)

A 01 N
C 12 N

The present search report has been drawn up for all claims

| Place of search | Date of completion of the search | Examiner |
|---|---|---|
| THE HAGUE | 10-12-1986 | DECORTE D. |